# EUROPEAN PATENT APPLICATION

(11) **EP 4 137 044 A1**
(43) Date of publication of application: **22.02.2023**
(21) Application number: 21191601.0
(22) Date of filing: 17.08.2021
(51) Int. Cl.: A61B 5/05, A61B 5/113, A61B 5/00

(54) **METHOD RELATED TO AN IN-VEHICLE INDUCTIVE SENSING APPARATUS**

(71) Applicant: Koninklijke Philips N.V., 5656 AG Eindhoven (NL)
(72) Inventor: STEUNEBRINK, Tim Patrick, 5656 AE Eindhoven (NL); WIJSHOFF, Ralph Wilhelm Christianus Gemma Rosa, 5656 AE Eindhoven (NL)
(74) Representative: Philips Intellectual Property & Standards

(57) **Abstract**

A method of calibrating an in-vehicle inductive sensing apparatus to the dimensions and/or posture of a user seated in the vehicle. An array of inductive sensing coils is mounted in a fixed position relative to a geometry of the seat in which the user is received. From the spatial pattern or distribution of inductive sensing signals, it is determined which subset of coils is positioned most appropriately, e.g. which is closest, to an anatomical body of interest. A biological measurement pertaining to the anatomical body of interest is computed using data from only the selected subset of coils.

## Description

### FIELD OF THE INVENTION

The present invention relates to a method relating to an in-vehicle inductive sensing apparatus.

### BACKGROUND OF THE INVENTION

Inductive sensing techniques permit measurement of volume and composition changes within a body.

An apparatus is used comprising at least one resonator circuit, the circuit comprising an inductive coil (preferably a single-loop coil) coupled to an oscillator, where the oscillator drives the coil with an alternating current. When the coil is placed over the body-to-be-sensed, eddy currents are induced in the body, which cause inducement of secondary electromagnetic fields. These interact with the primary field generated by the coil and alter electrical characteristics of the resonator circuit. The secondary field appears at the oscillator loop impedance as an additional complex impedance. Changes in this complex impedance give insight in the changing composition and volume of the body. This information can be used to extract biological parameters such as for example respiration and pulse.

The changes in the complex impedance can be measured in practice through measurement of consequent detuning of resonance characteristics of the resonator circuit (e.g. the natural oscillation frequency and oscillation amplitude). By measuring the detuning of these electrical characteristics, properties of bodily tissues and anatomical structures can be ascertained.

Further details pertaining to inductive sensing of this type can be found in WO 2018/127482 A1.

A growing field is that of in-vehicle monitoring of driver wellness. Driver wellness can be used for example to increase road safety by generating feedback prompting a driver to rest when needed. More generally, driving is a convenient time for obtaining informative biological information that may help a user manage their general health.

Vital signs, e.g. heart rate and respiration rate, provide a useful metric of certain aspects of driver wellness. Current techniques for vital sign measurement may either require additional hardware or demand deliberate steps to be performed by a user (e.g. putting on a watch with a PPG sensor). Even known in-vehicle sensing systems have constraints, e.g. requiring a user to place a portion of their body adjacent an integrated sensor in the case of in-vehicle heart rate or PPG sensors, e.g. integrated in the steering wheel.

### SUMMARY OF THE INVENTION

It is a proposal of the inventors to use inductive sensing technology to allow measurement of vital sign entirely passively (on the part of the driver), by integrating the sensors in the seat or an attachment placed over the seat or an attachment to the seat belt, or any other component which in normal use (during driving) has a defined (fixed) spatial relationship with the seat. This is a possible placement for inductive sensors since they are non-contact in nature, and can achieve the necessary electromagnetic interaction with the body through the material of the seat or attachment where necessary.

Integration of a sensor in or on the seat or to the seat belt allows for vital sign measurement at any time during driving, without the need for additional equipment to be placed on the user's body, or any deliberate action by the user, thereby improving convenience.

However, it has been recognized by the inventors that integrating the sensor array in a seat poses a challenge in determining the best subset of one or more coils to use to measure a target vital sign. For users of different heights or body dimensions, the placement of the relevant organs relative to the sensor array when the user is sat in the chair will differ. Also, the posture of the user is relevant for the same reason. In other words, if the position of the sensor array relative to the seat remains unchanged, different users will find the sensor array aligns with a different specific section of their body.

For example, to measure heart rate, the coil or subset of coils which is used to acquire data will need to be in proximity of the heart. The relative position of the heart to a car seat can be expected to differ to a substantive extent for a relatively short person compared to a relatively tall person sitting in the same seat. Furthermore, in addition to selecting the correct subset of one or more coils based on position, also optimal drive signal characteristics to achieve optimal excitation of the coil may need to be considered in order to optimize sensor performance depending upon the dimensions of the driver and/or posture of the driver.

An aim of embodiments of the present invention is to address one or more of the aboveidentified problems.

The invention is defined by the claims.

According to examples in accordance with an aspect of the invention, there is provided a method of using an in-vehicle inductive sensing apparatus,
wherein the inductive sensing apparatus comprises a plurality of inductor coils mounted in an array having a defined spatial arrangement relative to a user-engaging surface of a seat unit of the vehicle, the seat unit for receiving a user, and the inductor coils arranged to transmit/receive electromagnetic signals to the user's body when the user is received in the seat unit;
the method comprising:
   obtaining respective inductive sensing signals from each of the plurality of inductor coils;
   estimating a spatial arrangement of the inductor coil array relative to at least one anatomical landmark of the user's body based on a spatial pattern of the inductive sensing signals from the array of coils;
   deriving a biological measurement of the user based on a processing operation applied to inductive sensing signals from only a subset of the coils,
   wherein the subset is selected in dependence on the determined spatial arrangement of the inductor coil array relative to the body.

Embodiments of the proposed invention are based on locating the coil array relative to the body based on the pattern of signals observable across the extent of the array. The aim is for example to spatially calibrate or align the drive scheme for the array to the dimensions of the user's body so that the most appropriately positioned coils are used to obtain a set of biological measurements, e.g. vital signs.

The subset of coils may be selected based on a spatial positioning of the subset relative to an anatomical area of interest. The anatomical area of interest is one for example which is relevant for measuring the biological measurement.

The subset may be selected based on identifying a subset of the coils which is closest in its spatial positioning and geometric configuration to a pre-determined preferred spatial positioning and geometric configuration relative to the anatomical area of interest of the user. The subset may be selected for example which is closest in alignment to the anatomical area of interest.

With reference to the at least one biological measurement derived from the sensing signals, this may comprise a vital sign of the user, for example heart rate or respiration rate.

In some embodiments, the anatomical object of interest may be the heart and/or lungs. For example, to measure a heart rate, the coil, or group of coils with defined shape/size, which is closest in lateral alignment to the heart may be selected. For measurement of respiration rate, the group of coils closest to the lungs would be selected.

In some embodiments, the method may comprise obtaining a coil-body proximity measure for each coil based on processing of the sensing signal from each coil. The spatial arrangement of the array of inductor coils relative to the user's body may be determined based on a spatial pattern of tissue proximity measures from the array of inductive coils.

For example, based on signal amplitude or frequency, an estimate of proximity of the coil to the user's tissue can be determined. This can be used for example to sense which coils are solidly coupled with the body. This can be used to estimate the spatial distribution of the coils relative to certain body landmarks.

The coil-body proximity measure may in some cases be a binary variable, i.e. proximal vs not proximal or contact vs no contact. It may in other cases be a graded variable, e.g. a proximity distance measure.

In some embodiments, the method may comprise estimating which of the plurality of coils is aligned with an area of the body-facing surface which is directly in contact with the user's body based on the proximity measures for the array of inductive coils, and which coils are outside of this area.

For example, the estimating of the spatial arrangement of the coils relative to at least one landmark of the body may be based on the detection as to which group of coils is aligned with the area of the body in contact with the body-facing surface. The subset of coils would be selected from among the coils which are aligned with the area in contact with the user's body.

In some embodiments, the method may comprise estimating a dimension of the user's body based on a spatial extension of the identified set of coils aligned with the area of the user's body in contact with the seat unit surface.

For example a dimension of a user's upper body may be estimated, for example a height of the user's upper body. A lookup table or pre-defined function or algorithm may be used to relate the obtained inductive sensing signal data to the dimension measurement for example.

For example, the method may comprise detecting a level along the array at which there is a cut-off between coils in contact with the body and coils outside of the area in contact with the body. Depending upon the position of this cut off line along the relevant dimension of the array, a dimension of the part of the user's body which is in contact with that body-facing surface can be estimated, e.g. a height of the user's upper body.

In some embodiments, the determining of the proximity measure may comprise determining a difference in a frequency of the coil current compared to a reference frequency, and/or determining a difference in an amplitude of the coil current compared to a reference amplitude. For example the reference (null) measures may be obtained in a setup/initialization step at a time when the user is not seated in the seat unit. i.e. when the seat unit is empty. The null-measurement could be done when the driver unlocks the car. At this moment the driver is not present in the car. This will result in an accurate null-measurement which also takes into account environmental influences (such as temperature or position of the car seat). This is more accurate compared to for example instead using a factory calibration measurement or setting taken only once when the car is manufactured. When the driver starts the car or starts driving, a second measurement might be taken, since it is now certain that the driver is seated in the seat. This measurement can be compared to the null-measurement. Alternatively or additionally, if multiple seats in a car are equipped with sensing arrays, the array of an empty seat can be used to obtain the null-measurement. The (weight / pressure) sensors which are already present in the car seats to detect presence of a person can be used to determine whether a seat is empty and thus whether the seat can be used for a null-measurement. In this scenario, null-measurements from empty seats can also be determined during driving, to accommodate for changing climate conditions in the car.

In some embodiments, the method may comprise estimating a user posture and/or a user alertness based on detecting changes in the spatial arrangement/distribution of the coils relative to the user body, for example changes over the course of a driving session.

For example, this is preferably based on changes in the set of coils which are aligned with the area of the user's body in contact with the body-facing surface. If the user is slouching, a different sized and positioned area of the body-facing surface will be covered by the user's body compared to if they are sitting up straight for example. If for example the user begins to slouch, inductive sensors placed higher in the array will lose contact with the body. Derived alertness or posture information could be exported to a driver alertness detection system of the vehicle for example.

In some embodiments, the method may comprise performing the driving of the coils to generate the primary electromagnetic signals emitted into the body. This may comprise supplying each coil with an alternating drive signal, and wherein the obtaining of the sensing signal from each coil comprises, simultaneous to driving the coils, measuring variation of one or more electrical properties of the coil current as a function of time. This may be performed by a drive circuit and/or measurement circuit.

By way of example, the electrical properties may be amplitude and frequency. Detuning of the drive signal characteristics occurs responsive to the coil inductively coupling with tissue in the body. Measurement is based on sensing these variations in the drive signal characteristics. A variation signal may be extracted as the sensing signal for example.

The method further may, as mentioned above, comprise configuring one or more characteristics of the drive signals based on the determined spatial arrangement of the coils relative to the user's body. The one or more characteristics may include signal power and/or signal frequency.

In one advantageous set of embodiments, the method may comprise driving each of the plurality of coils with an alternating drive signal, and wherein the method comprises the following set of steps: setting an initial frequency and/or amplitude of the drive signals for the plurality of coils; obtaining an inductive sensing signal from each of the plurality of coils; determining an indicator of signal strength of each sensing signal, or determining a measure of coil-body proximity for each coil; and individually adjusting the frequency and/or amplitude of the drive signals for the plurality of coils based on the determined signal strength or proximity measure for each coil.

The aim is to for example increase the drive signal power or adjust the drive signal frequency in the event that signal strength is low or tissue proximity is low. Frequency of the drive signals affect the penetration depth of the resulting EM emissions. This embodiment may employ use of a predetermined adjustment algorithm or control program.

Various additional in-vehicle components or fittings may be utilized to aid in the determination of the arrangement of the coils relative to the user anatomy.

In some embodiments, the method may comprise receiving spatial sensing data of the user seated in the seat unit, from an optical or electromagnetic sensing device, and wherein the determining the spatial arrangement of the coils relative to the user is further based on use of the spatial sensing data. The spatial sensing data preferably provides a front-facing view of the user, enabling posture to be determined. The optical or electromagnetic sensing device may include a camera and/or a radar device for example. Furthermore, when using a camera, one may also obtain context information relevant for the optimization of the coil excitation. For instance, analysis of the camera images can show whether a person is wearing a coat or a t-shirt. The excitation of the coils might then be adjusted to the thickness of the layers of clothing between the sensors and the body.

Additionally or alternatively, in some embodiments, the method may comprise receiving pressure sensing data from at least one pressure sensor (e.g. a pressure sensor array) arranged for sensing a weight of a user when seated in the seating unit, and wherein the detecting of the spatial arrangement of the coils relative to the user is further based on use of the pressure sensing data. The pressure sensing data can be used to estimate a weight of the user, and this can be used to estimate a dimension of the part of the user's body which is engaged with the body facing surface of the seat, for example a height of the user's body.

Additionally or alternatively, in some embodiments, the method may comprise receiving data from a control unit of the vehicle, wherein the data includes at least one of: a steering wheel position, a seat position, a seatbelt extension length and/or position, and wherein the estimating of the spatial arrangement of the array of coils relative to the user is further based on use of the received data from the control unit of the vehicle. With regards to the position of the steering wheel and the seatbelt, this information can be used to estimate properties of the user's size, which can be used to optimize the selection of coils to obtain sensing data.

Additionally or alternatively, in accordance with one or more embodiments, the method may comprise obtaining inductive sensing signals from an inductor coil mounted to, e.g. integrated in, a belt portion of a seatbelt of the vehicle and wherein the estimating of the spatial arrangement of the inductor coil array relative to the user's body is further based on use of the sensing data from the seat-belt sensor coil. For example, a measurement/estimate may be made of the height of the seatbelt inductor coil relative to the seat by for instance integrating an additional radar or ultrasound sensor in the seatbelt inductive sensor. The radar or ultrasound measurement can be used prior to starting the at least one biological measurement (e.g. breathing and pulse measurements) with the inductive sensors to determine the height of the seatbelt sensor. Once the height of the seatbelt sensor has been determined, the additional radar or ultrasound sensor can be deactivated (switched to an off state) again to avoid any potential interference with the breathing and pulse measurements. Alternatively, an array of inductive sensors can be placed on the seatbelt and the array of inductive sensors on the seatbelt can be used to determine the location of the anatomical area of interest.

A further aspect of the invention provides a controller comprising one or more processors adapted to perform a method in accordance with any example or embodiment outlined above or described below, or in accordance with any claim of this application. The one or more processors are operable to perform the method when the controller is operatively coupled to an inductive sensing apparatus which comprises a plurality of inductor coils mounted in an array having a defined spatial arrangement relative to a user-engaging surface of a seat unit of a vehicle, the seat unit for receiving a user, and the inductor coils arranged to transmit/receive electromagnetic signals to the user's body when the user is received in the seat unit.

A further aspect of the invention also provides a computer program product comprising computer program code configured to cause a processor to execute the steps of the method according to any example or embodiment outlined above or described below.

A further aspect of the invention provides an in-vehicle inductive sensing system. The system comprises an inductive sensing apparatus comprising a plurality of inductive sensing coils mounted in an array having a defined spatial arrangement relative to a body-facing surface of a seat unit of the vehicle, the seat unit for receiving a user, and the inductive sensing coils arranged to transmit/receive electromagnetic signals via the body-facing surface to the user's body when the user is received in the seat unit, and the seat unit supporting the array in the defined spatial arrangement. The system further comprises a controller comprising one or more processors, operatively coupled with the inductive sensing apparatus.

The controller is adapted to perform at least the following steps: obtain respective inductive sensing signals from each of the plurality of inductor coils; estimate a spatial arrangement of the inductor coil array relative to at least one anatomical landmark of the user's body based on a spatial pattern of the inductive sensing signals from the array of coils; and derive a biological measurement of the user based on a processing operation applied to inductive sensing signals from only a subset of the coils. The subset of coils is selected in dependence on the determined spatial arrangement of the inductor coil array relative to the body.

In a preferred set of embodiments, each of the inductor coils is a single-loop inductor coil. A single-turn loop coil minimizes or even eliminates parasitic capacitive coupling between turns of the coil, which enables operation at higher frequencies, thereby increasing signal strength. It has been found that increasing the number of turns in the coils (to increase flux linkage) contributed no detectable improvement in sensing signal strength. Therefore, minimizing the number of turns of the coil is preferred to reduce the parasitic capacitances.

In some embodiments, the system comprises the seat unit and the array is fixedly mounted to the seat unit. In one example, the array of coils may be mounted beneath the body-facing surface, i.e. integrated in the body of the seat. In other examples, it may be integrated in an attachment which is adapted to couple to the chair, either fixedly or removably, such as a seat cover or pad. For example, the array could be integrated in a pad or attachment designed to be mounted to the seat unit, e.g. suspended from a head rest, or held in place with a strap.

In some embodiments, the body-facing surface may be a surface of a back portion of the seat unit, for supporting the user's back when seated in the seat unit. Additionally or alternatively, the body facing surface may comprise a surface of the seat portion of the seat unit.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows an example seat unit with an example array of inductor coils mounted relative thereto, as may be utilized in accordance with embodiments of the invention;
Fig. 2 shows a cross-section view of an example seat unit with a user received therein, showing how spatial arrangement of the coils relative to anatomical landmarks may differ for persons of different sizes and postures;
Fig. 3 shows an example processing arrangement for receiving and processing inductive sensing signals; and
Fig. 4 shows steps of an example method in accordance with one or more embodiments.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a method of calibrating an in-vehicle inductive sensing apparatus to the dimensions and/or posture of a user seated in the vehicle, for example a driver of the vehicle. An array of inductive sensing coils may be mounted in a fixed position relative to a geometry of the seat in which the user is received. There may be deliberate redundancy in terms of the number of inductive coils included in the sensor array. One subset of the coils of the inductive sensor array will be positioned most appropriately for obtaining sensing data for deriving a particular biological signal or measurement of interest, e.g. heart rate or respiration rate. The method proposes to identify from the spatial pattern or distribution of inductive sensing signals which subset of coils is positioned most appropriately, e.g. which is closest, and to decouple the signal data of the remainder of the coils from the measurement algorithms employed to derive the biological measurement of interest, or even to deactivate these coils. Additionally or alternatively, the coil drive signal characteristics can be adjusted in dependence upon the identified relative spatial arrangement of the coils with respect to the anatomy of the user, in order to calibrate the inductive sensing system to the coil-user positioning.

Thus, in at least some embodiments, the invention can be understood as seeking to use the inductive sensor array itself, and optionally additional sensors, to determine a length or height of the upper body relative to the car seat (and thus the coil array) and/or a posture of a driver in order to: (a) select the right coil(s) of the array and/or (b) to determine the optimal excitation settings of the coil(s). Selection of the right coil(s) and/or optimization of excitation settings can be performed using solely signal data from the inductive sensor array, or, optionally, additionally data from other sensors (preferably those already integrated in the hardware of the vehicle). These may include, without intending any limitation, one or more cameras, radar devices, weight sensors (e.g. in the seat), seat and/or steering wheel position settings, a seatbelt position and a length of the seatbelt withdrawn from the dispenser.

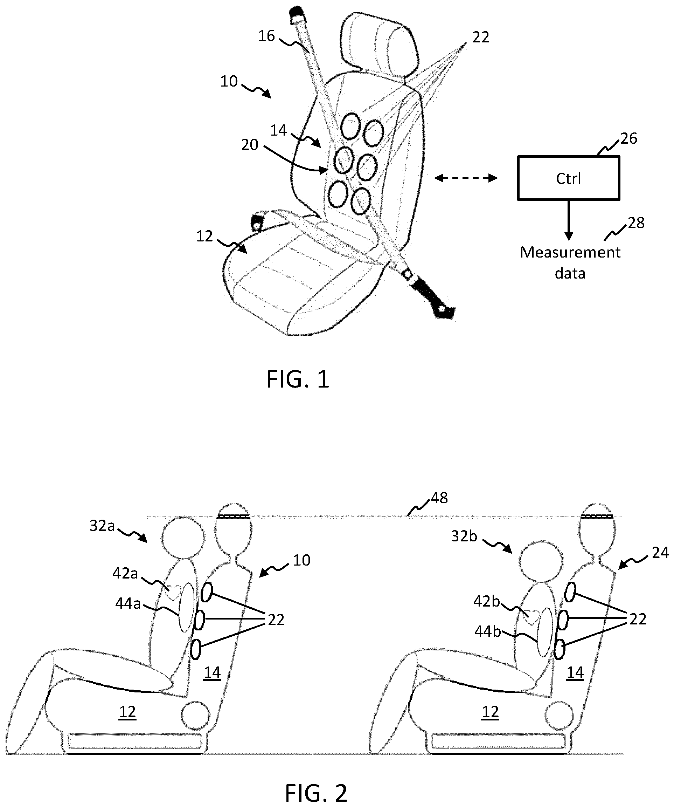

To help illustrate the concept embodied in the various aspects of the claimed invention, Fig. 1 shows an example in-vehicle inductive sensing apparatus installed in situ.

The inductive sensing apparatus comprises a plurality of inductor coils 22 mounted in an array 20 having a defined spatial arrangement relative to a user-engaging surface 14 of a seat unit 10 of the vehicle, the seat unit for receiving a user, and the inductor coils arranged to transmit/receive electromagnetic signals to the user's body when the user is received in the seat unit. Each inductor coil is preferably a single loop coil (or antenna).

In the illustrated example, the seat unit 10 is a vehicle seat, for example for receiving a driver's seat. It comprises a seat section 12, a back-rest section 14, and a headrest. The seatbelt 16 is also shown. The array 20 of coils is integrated beneath the user-facing surface of the back-rest portion of the seat unit in this example, so that when the user is sat in the seat, their back engages with this user-facing surface and can electromagnetically couple with at least a portion of the coils 22. Instead of being integrated in the seat, the coils could instead be integrated in an attachment or accessory such as a pad or seat cover which is fixed or fixable in a stable position relative to the seat, for example hanging from the backrest. Also, instead of, or in addition to, having an array of coils arranged to transmit-receive electromagnetic (EM) signals via the user-facing surface of the back-rest portion of the seat, an array of coils may be positioned to electromagnetically engage with the user's body via a different portion of the seat, e.g. a user-facing surface of the seat section 12.

In operation, each of the inductive sensing coils may be driven with an alternating current, thereby inducing an alternating electromagnetic (EM) field. The field generated by each coil may be referred to a transmitted/received electromagnetic signal, in the sense that it penetrates into the body of the user, and, through its interaction therewith, carries readable information within it by which biological measurements can be obtained. When the user is seated in the seat unit 10, the electromagnetic fields/signals generated by the coils extend into the body of the user and electromagnetically interact with structures of the body. The fields induce eddy currents in certain bodily tissues, which then in turn induce secondary EM field components. The secondary EM field components interact (superpose) with the primary EM field generated by the respective coil, and change therefore detectable electrical parameters of the oscillating current in the coil. In particular, the secondary EM fields change a complex impedance in the coil circuit. Changes in this complex impedance can be measured in real-time and give insight to the moment-to-moment variation in volume and geometry of internal structures that undergo cyclical motion (e.g. heart or lungs). This information can be used to extract (in a non-contact manner) biological parameters such as for example pulse or respiration rate.

Each coil may form an antenna component of a respective resonator circuit, the resonator circuit being driven by an oscillator, where the oscillator is preferably a dedicated oscillator, individually controllable, for each coil (to permit individuation in drive signal characteristics for each coil). Alternatively a common oscillator may be provided for the whole array of coils, but wherein resonance characteristics of each coil are individually tunable via an adjustable capacitor for instance.

The array 20 of inductor coils 22 is operatively coupled, for example through wired connection, to a controller 26. The controller receives the inductive sensing signals from each of the coils, and is adapted to derive at least one biological measurement of the user based on a processing operation (e.g. an algorithm or function) applied to inductive sensing signals from a subset of the coils.

The controller 26 is adapted to estimate a spatial arrangement of the inductor coil array 20 relative to at least one anatomical landmark of the user's body based on a spatial pattern of the inductive sensing signals from the array of coils. The controller is adapted to select a subset of the coils in dependence upon the determined spatial arrangement of the inductor coil array relative to the body. The controller is further adapted to derive the at least one biological measurement of the user based on application of the processing operation (e.g. algorithm or function) applied to inductive sensing signals from only the selected subset of the coils. The controller generates a data output 28, i.e. a data signal, indicative of the obtained biological measurement information. This may be transmitted to a further computing device, or to a datastore, e.g. to a cloud-based datastore, or to a user's mobile computing device, or to a further computing or processing unit comprised by the vehicle. The measurement information may be displayed using a user interface installed in the vehicle in some examples.

The anatomical landmark may be an anatomical structure of interest, for example an organ. It may be an anatomical structure to which the biological measurement pertains, e.g. the heart for the heart rate, or the lungs for the respiration rate. Alternatively, the anatomical landmark could be any other reference point on the body which may act as a convenient reference marker for measuring relative positioning of the body relative to the coil array, e.g. the top of the head or base of the spine or any other point.

The driving of the coils, and the extraction of the biological measurements will be discussed in greater detail later.

However, first, the problem of selecting the right subset of coil(s) 22 of the inductive sensing array 20 will be discussed in more detail, following which a number of preferred embodiments will be discussed for implementing the selection and for configuring optimal excitation settings.

Fig. 2 shows the seat unit 10 and inductive sensing apparatus of Fig. 1 from an elevation cross-sectional view, and shows the seat unit with two different users 32a, 32b received in the seat unit, the users different in height. Line 48 shows the height level of the top of the head of the first user 32a depicted in Fig. 2 (left), and the disparity in height compared with the second user 32b can be seen in Fig. 2 (right).

By way of illustration, the problem addressed by the invention will be discussed with reference to embodiments in which measurement of a vital sign such as heart rate and/or respiration rate is the aim. However, the invention is not limited to only these biological measurements. Others may include the measurement of body tissue composition (e.g. including fat and muscle proportions, and/or detection of hemorrhaging), lung capacity, volume of air inhaled/exhaled, or an estimate of cardiac output from heart measurements, which measurements possibly require the combination with a null measurement (described elsewhere in this disclosure).

In order to measure vital signs with an inductive sensor, the sensor needs to be close to the lungs or heart when measuring respiration or pulse respectively. If the plurality of inductive sensors (sensor array) is fixed in position relative to the seat, then when persons of different upper body length seat themselves in the seat, the position of the heart and lungs relative to the sensor array will differ from person to person. This is shown in Fig. 2 which schematically shows the position of the first user's 32a heart 42a and lungs 44a and the second user's 32b heart 42b and lungs 44b. It can be clearly seen that the heart 42a of the taller person 32a (left) is closest to the subset of sensors consisting of the top row of sensors of the illustrative array of sensors 22, and the lungs 44a closest to the subset of sensors consisting of the top and middle row of sensors. In contrast, for the shorter person 32b (right) the heart 42b is closest to the middle row of sensors and the lungs 44b closest to the subset comprising the bottom and middle rows of sensors. As a result of this, different subsets of coils need to be selected for deriving heart rate and deriving respiration rate, and wherein these subsets also differ between persons of differing upper body length relative to the sensor array position.

Thus, embodiments of the invention may comprise selecting the subset of coils based on identifying the subset of the coils which is closest in its spatial positioning and geometric configuration to a pre-determined preferred spatial positioning and geometric configuration relative to the anatomical area of interest of the user. By way of example, the subset may be selected which is closest in alignment to the anatomical area of interest.

For example, the controller may access a stored dataset which lists or catalogues, for each of a plurality of different anatomical areas of interest, a preferred geometric configuration of the subset of coils and a preferred spatial positioning of the subset of coils. With regards to the geometric configuration, this may comprise a preferred number of coils to use, and a preferred shape or pattern of this subset of coils. For instance, for sensing the lungs for respiration rate, a larger sensing area may be preferred, e.g. using a subset of two or more coils. This may comprise a spatially continuous group of active coils, or a pattern of coils which is more spread out, e.g. every other coil is active within an area closest to the lungs. For the heart, a smaller sensing area may be preferred, e.g. using just a single coil.

If a preferred geometric configuration and spatial positioning for the subset of coils is known, the task of the processor is then to detect a metric indicative of relative positioning of the array of coils relative to the body anatomy of the user, and then to select, based upon this information, the subset of coils which conforms most closely to the preferred spatial positioning and geometric configuration.

For determining the relative spatial arrangement of the array of coils with respect to the user's anatomy, in one set of embodiments, sensing signals from the coil array alone may be used.

For example, the controller 26 may be configured to perform a calibration phase when a user first seats themselves in the seat. For example, this might be triggered upon starting of the vehicle ignition, or might be manually triggered by the user with a user control. The controller receives an initial set of sensing signals from the array of inductive sensors and, based on a spatial pattern or distribution of the signal data, and using a pre-configured algorithm or function or look-up table, determines calibration information indicative of likely relative position of the array with respect to the user anatomy, e.g. with respect to one or more anatomical landmarks such as the heart or lungs. The subset of coils is then chosen, and the controller proceeds to a subsequent operational phase, in which inductive sensing signals from only the chosen subset of coils is obtained and this is used to derive the one or more biological measurements. The other coils may still be driven, but wherein their signal data is not taken into account in the measurement algorithms. Alternatively, the coils not forming part of the selected subset may be deactivated (not driven), which may save system resource or reduce interference with the coils which are part of the selected subset. They could be reactivated at a later stage, for example if the calibration phase is repeated.

With regards to the calibration phase, this can be done in different ways.

In at least one set of embodiments, the method comprises obtaining a coil-body proximity measure for each of the plurality of coils based on processing of the sensing signal from each coil (obtained in the calibration phase).

The presence of a body in proximity of the coil loop generates a significant detuning of resonance characteristics of the resonator circuit of which each coil is a part. Thus it is possible to sense which coils are in proximity to bodily tissue, and which are not. For example, referring to the example depicted in Fig. 2, it is clear that tissue is present in proximity of the top row of sensors for the first (taller) user 32a on the left, but not for the second (shorter) user 32b on the right. The middle and bottom rows of sensors are in proximity of tissue for both subjects. This means that the resonator circuits will detune significantly for each of the middle and bottom rows of sensors for both users, but the top row of sensors will only detune for the taller user. Using this result, the relative positioning of the sensor coil array with respect to the user anatomy can be estimated. For example, the position of the shoulders could be determined and with this measurement an estimate can be made of the position of the heart in order to select the right coil(s) for pulse measurements. Likewise, the position of the lungs can be determined and the right coil(s) for respiration measurements can be selected. This approach assumes a certain standard distribution of the heart and lungs relative to the length of the upper body. This information may be stored in a local datastore explicitly, or may be implicitly programmed in a coil selection program or algorithm utilized by the controller for example.

To sense tissue proximity for a given coil, the obtained signal for each coil may be compared with a baseline null signal value, known to be associated with absence of tissue in proximity of the coil. For example, the null-measurement for each coil may be obtained before a user seats themselves in the seat. For example, it might be captured by the controller as the user unlocks the vehicle, so that the user is known not be in the vehicle, but is captured at a moment in time close to when the user will be seated. This will result in an accurate null-measurement which takes into account any environmental influences (such as temperature or position of the car seat) in the car, as compared to a 'factory calibration measurement' taken only once when the car is manufactured. When the driver starts the car or starts driving, the calibration operation might then be triggered, in which a further set of inductive sensing signals are obtained from the array of coils, since it is now certain that the driver is seated. This measurement can be compared to the null-measurement to determine the coil-body proximity measure for each coil.

This general approach of obtaining a null-measurement and a second (calibration) measurement when starting the car can be used in any embodiment described in this disclosure.

The null measurement can also be used when deriving the biological measurement from the inductive sensor data obtained from the chosen subset of coils. For example, without the null measurement, the signals from the coils can still be used to track relative progression/variation in the relevant anatomical structure, e.g. pulsing of the heart, inflation/deflation of the lungs, and thus from this alone a pulse rate and respiration rate can be obtained. With the added use of a null-measurement, this might allow more absolute measurement data to be obtained, e.g. lung capacity, or volume of air inhaled/exhaled, or a rough estimate of cardiac output from heart measurements.

In some examples, multiple seats in the vehicle may comprise respective inductive sensing apparatuses for performing biological sensing of users received therein. In these cases, optionally, instead of obtaining the null measurement from the inductive sensor array in the primary seat at a time that the user is not yet seated in the seat unit, the null measurement could be obtained from an inductive sensor array of a different seat unit in the same vehicle, the different seat unit being empty. For example, each seat unit in the vehicle which is installed with inductive sensors may incorporate a weight or pressure sensor for sensing a weight of a person received in the seat. The weight or pressure sensor readings can therefore be used to detect which seats are occupied and which are empty. In this scenario, null-measurements might be acquired from one or more empty seats, enabling for example updating of the null-measurement throughout driving, permitting changing environmental conditions in the vehicle, e.g. climate conditions, to be accounted for in the processing performed by the controller.

Returning to the selection of the subset of coils, during the calibration operation, a respective coil-body proximity measure is obtained for each coil. In some examples, the coil-body proximity measure may be a binary variable, i.e. proximal vs not proximal or contact vs no contact. In other examples, it could be a graduated variable. The spatial arrangement of the array of inductor coils relative to the user's body may then be determined based on a spatial pattern of the tissue proximity measures from the array of inductor coils. In other words, it can be determined which coils are in close contact with the body, and thus a measure of how the user's body is situated relative to the coil array, either due to height or posture, can be derived.

In some examples, the method may comprise estimating which of the plurality of coils is aligned with an area of the body-facing surface which is directly in contact with the user's body based on the proximity measures for the array of inductive coils, and which coils are outside of this area. In other words, based on the proximity measures, it can be determined the area of the seat surface that the user's back (or other body region) is in direct contact with. If the proximity measures are each binary, this would simply be the area over which all of the sensor coils show positive proximity measures, and excluding the area outside of this where the proximity measures are zero.

The subset of coils would be selected from among the coils which are aligned with the area in contact with the user's body. Based on the detected area in contact with the user's body, a preprogrammed algorithm may be operable to determine the subset of coils to use for obtaining sensor data for a particular biological measurement of interest, since this area is implicitly indicative of relative position of the user's anatomy with the sensor coil array.

In some examples, the method may comprise estimating a dimension of the user's body based on a spatial extension of the identified set of coils aligned with the area of the user's body in contact with the seat unit surface. For example a dimension of a user's upper body may be estimated, for example a height of the user's upper body. For example, the method may comprise detecting a level along the array at which there is a cut-off between coils in contact with the body and coils outside of the area in contact with the body. Depending upon the position of this cut off line along the relevant dimension of the array, and based on knowledge of a spatial geometry of the array, a dimension of the part of the user's body which is in contact with that body-facing surface can be estimated, e.g. a height of the user's upper body.

In any of the above-described examples, the relative positioning of the user's body relative to the coil array which is detected could be representative simply of dimensions of the user's body, and/or could be reflective of user posture. The same measurement approach described will naturally capture both factors. In some embodiments, since user posture can be expected to change over time throughout driving, both for the driver and passengers, the calibration operation (in which the spatial arrangement of the inductor coil array relative to the body is determined) may be repeated multiple times throughout a driving session. It may be repeated at regular intervals, or re-calibration may be triggered by one or more data inputs to the controller, e.g. inputs from other sensors (e.g. cameras, or weight sensors detecting shift of weight), or based on systematic changes in the inductive sensing signals themselves. In the latter case for example, the proximity measures can be reacquired at any arbitrary time to detect whether the area of the seat with which the user's body is in contact has changed, and adjustment of the selected subset of sensor coils performed responsive to such a detected change.

In some embodiments, a posture of the user may be explicitly monitored and tracked using the sensor array. This information may not only be used to adjust the subset of sensors used to compute the biological measurement, but may also be used to estimate changes in driver alertness. For example, user posture and/or a user alertness may be detected based on detecting changes in the spatial arrangement/distribution of the coils relative to the user body. For example, this is preferably based on changes in the set of coils which are aligned with the area of the user's body in contact with the body-facing surface. If somebody is alert and fit, the person will sit up straight. If somebody gets tired, the person may sit less straight. If the user is slouching, a different sized and positioned area of the body-facing surface will be covered by the user's body compared to if they are sitting up straight for example. If for example the user begins to slouch, inductive sensors placed higher in the array will lose contact with the body. By comparing the inductive sensing signals of each coil with the respective null-measurements (as explained in the previous embodiment), it can be determined which inductive sensors are in contact with the body and when the highest placed inductive sensors in the array start losing contact with the body. If higher-placed inductive sensors start losing contact for a prolonged period of time, this can indicate that a driver is losing alertness. This information could be exported to a driver alertness detection system of the vehicle for example.

As mentioned above, not only the subset of coils selected for obtaining the biological measurement, but also drive characteristics of the subset of coils, individually or collectively, may be configured based on the determined spatial arrangement of the coils relative to the user's body. By way of example, the one or more characteristics which are adjusted may include signal power and/or signal frequency. It is known that signal power affects penetration depth of signals. However, it is also the case that signal frequency affects penetration depth.

This dependency can be utilized to adjust the signal frequency and/or power to improve, for instance, motion robustness. Different drivers with different postures will yield a different response from each inductive sensor. This can also influence motion robustness since the sensitivity of the sensor is a function of distance and varies for different tissues in proximity to the coil. For example, having a very muscular person in the chair results in a different sensitivity at a certain depth in the body than a less athletic person. Also, persons with a thicker fat layer have the lungs and heart located further away from the sensors. To be sensitive at the desired depth and to be able to measure sufficiently deep inside the body, excitation of the inductive coils is preferably optimized from person to person. This may include the excitation frequency, or frequencies for multi-frequency systems, and the radiated power.

Thus, the excitation characteristics of the inductive coils may be adjusted based on the determined spatial arrangement of the coils relative to the user's body.

By way of example, a specific implementation may run as follows. The method may comprise driving each of the plurality of coils with an alternating drive signal, and wherein the method comprises:
setting an initial frequency and/or amplitude of the drive signals for the plurality of coils;
obtaining inductive sensing signals from at least an initial subset of the plurality of coils;
determining an indicator of signal strength, e.g. signal-to-noise, of the sensing signals for the at least initial subset of coils, or determining a measure of coil-body proximity for each coil; and
individually adjusting the frequency and/or amplitude of the drive signals for the plurality of coils based on the determined signal strength or proximity measure for each coil.

The aim is to for example increase the drive signal power or adjust the drive signal frequency in the event that signal strength is low or tissue proximity is low.

In accordance with one or more embodiments, the at least initial subset may be chosen to be the lower-most row of coils in the coil array, since this is most likely to always be in close coupling with the user's tissue. Furthermore, in some examples, the inductive signals obtained from the at least initial subset and used to adjust the drive characteristics of the coils may be averaged over a longer window of time to filter out any transient fluctuations. For example, taking the average value of the inductive sensor signal, e.g. the oscillation frequency, damping value, and/or amplitude, over a longer period of time filters out errors due to motion and vital signs. The resulting DC-levels in the inductive sensor signals, such as the oscillation frequency and damping, resulting from the inductive sensors from the bottom row of coils, are indicative of the coupling between the sensors and the tissue for a specific person. This DC-level could then be used to optimize coil excitation. In some examples, the sensing signals obtained from the at least initial subset of coils and used to optimize the excitation characteristics may be the same sensing signals used in normal operation to obtain the biological measurements, but for example simply averaged over a certain window of time, and/or are just the signals from one select group of the coils (e.g. just the bottom row).

In accordance with one or more embodiments, the inductive sensing signals from the inductive sensing apparatus may be used to estimate a posture of the user. This can be achieved in general based on sensing which of the array of coils is aligned (i.e. facing in contact with) the body of the user, and which is not. This is described in more detail elsewhere in this disclosure. For the purposes of sensing posture, or changes in posture, it may be beneficial to obtain a baseline measure of which of the array of coils is aligned with an area of the body-facing surface which is directly in contact with the user's body, and which are not. This baseline could be obtained based on averaging over sensing signals acquired over a (relatively longer) window of time, to filter transient fluctuations or brief adjustments in position.

Various additional in-vehicle components or fittings may be utilized to aid in the assessment of the positioning and size of the user.

In some embodiments, the method may comprise receiving spatial sensing data of the user seated in the seat unit, from an optical or electromagnetic sensing device, and wherein the determining the spatial arrangement of the coils relative to the user is further based on use of the spatial sensing data. The spatial sensing data preferably provides a front view of the user, enabling posture to be determined. The optical or electromagnetic sensing device may include a camera and/or a radar device for example.

For example, an in-car camera system may be utilized. A camera may for instance be integrated in the rear-view mirror or replace the rear-view mirror. In this example, analysis of the camera images permits detection of a length or height of the upper body of the person relative to the sensor array and thus which sensors can be used best for pulse and breathing measurements. Also, analysis of the camera images may permit posture of the driver to be determined, which information can be used to configure the system in the manner discussed above.

Furthermore, analysis of image data from a camera may be used to obtain further contextual information, such as clothing of the user. For example, depending upon whether a person is wearing a coat or a t-shirt, the drive signal characteristics of the coils may then be adjusted in dependence upon the thickness of the layers of clothing between the sensors and the body.

Additionally or alternatively, in some embodiments, the method may comprise receiving pressure sensing data from a pressure sensor arranged for sensing a weight of a user when seated in the seating unit, and wherein the detecting of the spatial arrangement of the coils relative to the user is further based on use of the pressure sensing data. The pressure sensing data can be used to estimate a weight of the user, and this can be used to estimate a dimension of the part of the user's body which is engaged with the body facing surface, for example a height of the user's body.

Often pressure sensors are already integrated in vehicle seats, as part of an in-vehicle safety system, e.g. to sense whether to activate an airbag. The same sensor could be utilized to estimate the weight of the user and use this information as part of estimating the relative spatial arrangement of the coils with respect to the body. If multiple pressure sensors are available, for example in the back support section 14 as well as in the seat section 12, data from the plurality of sensors may be used.

Additionally or alternatively, in some embodiments, the method may comprise receiving data from a control unit of the vehicle, wherein the data includes at least one of: a steering wheel position, a seat position setting.

An estimate of the length of the upper body of the driver can be inferred from the position and settings of the seat and the steering wheel. Taller persons will have the seat positioned at a larger distance from the steering wheel than shorter persons. Based on assumptions relating to average human proportions, e.g., between the length of the legs and the length of the upper body, it is possible to determine which coils are best to use for respiration and pulse measurements based on the distance between the seat and the steering wheel and the height of the steering wheel.

The settings of the seat and steering wheel position are often stored in the car key, so that when the car is opened with a specific key, the seat and steering wheel automatically take on the required position. In this scenario, the settings of the inductive sensor array might also be stored in the car key, so the inductive sensor array can be automatically configured upon door unlocking or upon insertion of the key in the ignition. Here, the stored settings of the inductive sensor array comprise (1) which sensors to use for respiratory measurements and which for pulse measurements and (2) the optimal excitation of the coils, as this information would already have been acquired and stored based in previous measurement sessions.

Additionally or alternatively, in accordance with one or more embodiments, the method may comprise receiving data from a control unit of the vehicle, wherein the data includes a seatbelt extension length and/or position, and wherein the estimating of the spatial arrangement of the array of coils relative to the user is further based on use of the received data from the control unit of the vehicle.

The seatbelt extension length means the length of seatbelt which has been pulled out by the user from the dispenser. It may be measured for example based on a sensor integrated in the seatbelt dispenser, which is operable to output a metric indicative of length of seatbelt withdrawn therefrom. The seatbelt extension amount is of course related to the girth of the user, and thus can be used as one information source informing the determination of the arrangement of the array of coils relative to the anatomy of the user. Since the seatbelt is anchored at two fixed points (left and right side of the seat), these known dimensions might be used to estimate the upper body length and posture of a driver. Coil selection and excitation can then be adjusted accordingly.

Additionally or alternatively, in accordance with one or more embodiments, the method may comprise obtaining inductive sensing signals from an inductor coil integrated in a belt portion of a seatbelt of the vehicle and wherein the estimating of the spatial arrangement of inductor coil array in the seat relative to the user's body is further based on use of the sensing data from the seat-belt inductor coil. In some examples, the inductor coil in the seatbelt has a fixed position along the length of the seat-belt, in which case an estimate of its position relative to the body could be used to estimate how close it is to the heart of the user, from which could be derived estimations about the general dimensions of the user (based on knowledge about the geometry of the seatbelt). This can then be used to determine spatial positioning of the user relative to the coil array in the seat. The position of the inductor coil relative to the body could be determined for example using camera or radar data of a sensor module housing the seatbelt inductive coil. Alternatively, a sensor module housing the seatbelt coil may further house one or more additional sensors, e.g. a radar sensing device or an ultrasound sensor, and wherein the position of the seatbelt inductor coils relative to the body could be determined based on data from these additional sensors. The additional sensors may be activated only during the calibration phase in which the positioning of the inductor coil array is being determined and the subset of coils selected. Subsequently, the additional sensors may be deactivated to avoid interference with the biological measurement acquisition.

Alternatively, the position of the inductor coil relative to the body could be determined based on properties of the sensing signal from the coil itself, e.g. signal strength or signal-to-noise ratio (SNR) for example.

In further examples, the sensor coil in the seatbelt may be moveable along the belt of the seat-belt (e.g. integrated in a sensor module which is slidable along the belt) to permit a user to manually position the sensor over their heart. Then, the manually adjusted position of the sensor coil along the belt could be detected with an integrated sensor, and used as a metric to estimate the dimensional information about the user, and from this information an estimate of the positioning of the coil array relative to the user's body determined.

In further examples, an array of inductive sensors may be provided along the belt portion of the seatbelt and wherein inductive sensing signals from this array of inductive sensors used at least in part to determine the location of an anatomical landmark, e.g. the shoulders, based on an equivalent method to that described above in relation to the array of sensors in or on the seat unit. Based on the detected location of the shoulders and on a determined angle of the seatbelt, the subset of the inductor coils in the seat which are to be used for biological measurements (e.g. heart rate) can be determined. The angle of the seatbelt can be determined based on knowledge of the two fixed anchor points of the seatbelt (as mentioned above), and based on the length of seatbelt which has been withdrawn from the dispenser.

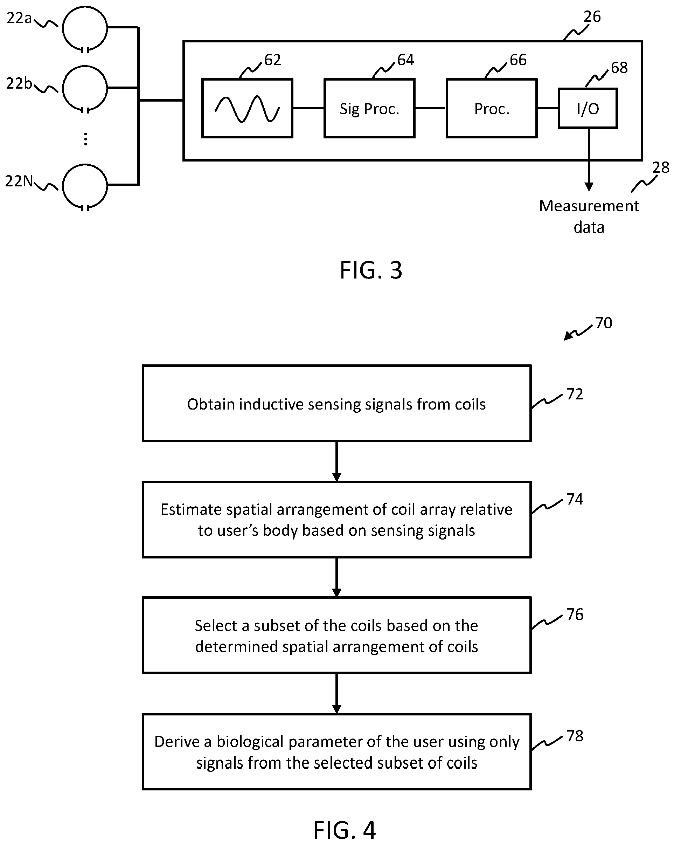

The technical problem addressed by aspects of the claimed invention primarily pertains to calibration of the inductive sensing system to the position and dimensions of the user received in the seat unit. The hardware for driving the coils and extracting signal data therefrom is not the primary focus, and may be provided as part of the claimed system, or may be separate to the claimed system, and wherein the system merely interacts therewith. In either case, to further clarify and exemplify embodiments of the invention, principles underlying the driving of the coils and the extracting of measurement data therefrom will now be discussed in more detail with reference to Fig. 3.

Fig. 3 shows a block diagram of components of an example inductive sensing system including an array of N inductive sensing coils 22a-22N, and a controller 26 operatively coupled with the inductive sensor array, adapted to simultaneously drive the coils to generate electromagnetic signals and to detect returned signal information from the body.

Each coil forms part of a respective resonator circuit which has a natural resonance frequency and natural oscillation amplitude, where these depend upon the capacitance of a capacitor included in the resonator circuit.

The controller 26 comprises at least one oscillator 62 for generating an AC drive signal for supply to the inductive coils 22 coupled to the controller 26 to drive generation of electromagnetic signals for transmission into the body. In some examples, a respective oscillator may be provided for driving each respective inductive coil, thereby permitting different individual configuration of drive signal characteristics for each individual coil. In other examples, a common oscillator 62 may be used, but wherein the drive signal characteristics for each coil may be individually adjusted based on adjusting resonance properties of the resonator circuit to which each coil belongs, for example adjusting a configurable capacitor in each resonator circuit.

The controller 26 also comprises a signal processing module 64 which is adapted to monitor electrical characteristics of a current in each of the resonator circuits of the plurality of inductive coils, and to detect changes in the natural resonance characteristics of each respective resonator circuit. For example, the signal processing module 64 may be adapted to detect changes in the resonance frequency of each resonator circuit and/or changes in the natural oscillating amplitude of each resonator circuit. As discussed above, when the primary electromagnetic field generated by a given inductive coil induces eddy currents in the body, thereby stimulating secondary electromagnetic fields, the secondary fields interact with the primary field and this results in detectable changes in the resonance characteristics of the coil circuit, i.e. the tuning of the coil resonance characteristics. By monitoring the changes in these characteristics as a function of time, real-time variations in volumes and positions of internal anatomical structures can be detected. The output of the signal processing module 64 may be a respective sensing signal for each inductor coil, where the sensing signal is indicative of the variation of the relevant one or more monitored signal characteristics of each inductor coil circuit. This provides an effective proxy measure of anatomical body volume and/or position changes. In some examples, the signal processing module may in fact comprise a plurality of signal processing components, including a respective signal processing component for each of the inductor coils 22.

The controller 26 preferably further comprises one or more processors 66 adapted to receive the respective sensing signals extracted from the raw signal data obtained from each coil, these being received from the signal processing module 64. The one or more processors 66 are adapted to determine one or more biological measurements, such as one or more vital signs, based on the received sensing signals for each coil. The one or more processors 66 are further adapted to perform the calibration operation discussed in detail above, wherein the subset of inductor coils is selected, and exclusively the data therefrom used for determining the one or more biological measurements. The processor may perform other functions such as controlling the drive signal characteristics, such as the frequency and/or amplitude of the alternating signal supplied to each coil by the one or more oscillators 62. The one or more processors 66 are communicatively coupled with an input/output (I/O) 68, and a data signal carrying a representation of the generated biological measurement data is output from the controller 26 via the I/O.

Computing the biological measurement based on the sensing data may be based on use by the one or more processors 66 of a pre-determined computation function or algorithm which is adapted to receive inductive sensing signal data and to output a biological measurement. For example, it may receive recurrent data samples from the subset of coils in real time, and may be adapted to detect from the data values when each respective movement cycle of the relevant anatomical structure begins and ends, and from this, and using an internal timer or using time stamps of the data sample, may compute a real-time heart rate or respiration rate.

Fig. 4 outlines the basic steps of an example method 70 according to one or more embodiments of the invention. The method is a method of using an in-vehicle inductive sensing apparatus, wherein the inductive sensing apparatus comprises a plurality of inductor coils mounted in an array having a defined spatial arrangement relative to a user-engaging surface of a seat unit of the vehicle, the seat unit for receiving a user, and the inductor coils arranged to transmit/receive electromagnetic signals to the user's body when the user is received in the seat unit.

The method comprises obtaining 72 respective inductive sensing signals from each of the plurality of inductor coils. The method further comprises estimating 74 a spatial arrangement of the inductor coil array relative to at least one anatomical landmark of the user's body based on a spatial pattern of the inductive sensing signals from the array of coils. The method further comprises selecting 76 a subset of the inductor coils in dependence upon the determined spatial arrangement of the inductor coil array relative to the body. The method further comprises deriving 78 a biological measurement of the user based on a processing operation applied to inductive sensing signals from only the selected subset of the coils.

As discussed above, the system makes use of one or more processors to perform the data processing. The processor can be implemented in numerous ways, with software and/or hardware, to perform the various functions required. The processor typically employs one or more microprocessors that may be programmed using software (e.g., microcode) to perform the required functions. The processor may be implemented as a combination of dedicated hardware to perform some functions and one or more programmed microprocessors and associated circuitry to perform other functions.

Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

A single processor or other unit may fulfill the functions of several items recited in the claims.

The mere fact that certain measures are recited in mutually different dependent claims does not indicate that a combination of these measures cannot be used to advantage.

A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to".

Any reference signs in the claims should not be construed as limiting the scope.

## Claims

1. A method of using an in-vehicle inductive sensing apparatus,
wherein the inductive sensing apparatus comprises a plurality of inductor coils mounted in an array having a defined spatial arrangement relative to a user-engaging surface of a seat unit of the vehicle, the seat unit for receiving a user, and the inductor coils arranged to transmit/receive electromagnetic signals to the user's body when the user is received in the seat unit;
the method comprising:
obtaining respective inductive sensing signals from each of the plurality of inductor coils;
estimating a spatial arrangement of the inductor coil array relative to at least one anatomical landmark of the user's body based on a spatial pattern of the inductive sensing signals from the array of coils; and
deriving a biological measurement of the user based on a processing operation applied to inductive sensing signals from only a subset of the coils,
wherein the subset is selected in dependence on the determined spatial arrangement of the inductor coil array relative to the body.

2. A method as claimed in claim 1, wherein the subset is selected based on a spatial positioning of the subset relative to an anatomical area of interest.

3. A method as claimed in claim 2, wherein the subset is selected based on:
identifying a subset of the coils which is closest in its spatial positioning and geometric configuration to a pre-determined preferred spatial positioning and geometric configuration relative to the anatomical area of interest of the user, and
optionally wherein the subset is selected which is closest in alignment to the anatomical area of interest.

4. A method as claimed in any of claims 1-3, wherein the method comprises obtaining a coil-body proximity measure for each coil based on processing of the sensing signal from each coil.

5. A method as claimed in claim 4, wherein the spatial arrangement of the array of inductor coils relative to the user's body is determined based on a spatial pattern of tissue proximity measures from the array of inductive coils.

6. A method as claimed in claim 5, wherein the method comprises:
estimating which of the plurality of coils is aligned with an area of the body-facing surface which is directly in contact with the user's body based on the proximity measures for the array of inductive coils, and which coils are outside of this area, and
optionally wherein the method comprises estimating a dimension of the user's body based on a spatial extension of the identified set of coils aligned with the area of the user's body in contact with the seat unit surface.

7. A method as claimed in any of claims 1-6, wherein the method comprises performing driving of the coils by supplying each coil with an alternating drive signal, and wherein the obtaining of the sensing signal from each coil comprises, simultaneous to driving the coils, measuring variation of one or more electrical properties of the coil current as a function of time.

8. A method as claimed in claim 7, wherein the method further comprises configuring one or more characteristics of the drive signals based on the determined spatial arrangement of the coils relative to the user's body, and preferably wherein the one or more characteristics include signal power and/or signal frequency.

9. A method as claimed in any of claims 1-8, wherein the biological measurement is a vital sign of the user, for example heart rate or respiration rate.

10. A method as claimed in any of claims 1-9, wherein the method comprises:
receiving spatial sensing data of the user seated in the seat unit, from an optical or electromagnetic sensing device, and wherein the determining the spatial arrangement of the coils relative to the user is further based on use of the spatial sensing data; and/or
receiving pressure sensing data from a pressure sensor arranged for sensing a weight of a user when seated in the seating unit, and wherein the detecting of the spatial arrangement of the coils relative to the user is further based on use of the pressure sensing data; and/or
receiving data from a control unit of the vehicle, wherein the data includes at least one of: a steering wheel position, a seat position setting, a seatbelt extension length and/or position, and wherein the estimating of the spatial arrangement of the array of coils relative to the user is further based on use of the received data from the control unit of the vehicle.

11. A method as claimed in any of claims 1-10, wherein the method further comprises obtaining inductive sensing signals from an inductor coil mounted to a belt portion of a seatbelt of the vehicle, and wherein the estimating of the spatial arrangement of the inductor coil array relative to the anatomical landmark is further based on use of the inductive sensing signals from the seatbelt inductor coil.

12. A controller comprising one or more processors adapted to perform the method according to any of claims 1-11 when the controller is operatively coupled to an inductive sensing apparatus which comprises a plurality of inductor coils mounted in an array having a defined spatial arrangement relative to a user-engaging surface of a seat unit of the vehicle, the seat unit for receiving a user, and the inductor coils arranged to transmit/receive electromagnetic signals to the user's body when the user is received in the seat unit.

13. A computer program product comprising code configured to cause a processor to execute the steps of the method of any of claims 1-11 when the processor is operatively coupled to an inductive sensing apparatus which comprises a plurality of inductor coils mounted in an array having a defined spatial arrangement relative to a user-engaging surface of a seat unit of the vehicle, the seat unit for receiving a user, and the inductor coils arranged to transmit/receive electromagnetic signals to the user's body when the user is received in the seat unit.

14. An in-vehicle inductive sensing system comprising:
an inductive sensing apparatus comprising a plurality of inductive sensing coils mounted in an array having a defined spatial arrangement relative to a body-facing surface of a seat unit of the vehicle, the seat unit for receiving a user, and the inductive sensing coils arranged to transmit/receive electromagnetic signals via the body-facing surface to the user's body when the user is received in the seat unit, and the seat unit supporting the array in the defined spatial arrangement; and
a controller comprising one or more processors, operatively coupled with the inductive sensing apparatus, adapted to:
obtain respective inductive sensing signals from each of the plurality of inductor coils;
estimate a spatial arrangement of the inductor coil array relative to at least one anatomical landmark of the user's body based on a spatial pattern of the inductive sensing signals from the array of coils; and
derive a biological measurement of the user based on a processing operation applied to inductive sensing signals from only a subset of the coils,
wherein the subset is selected in dependence on the determined spatial arrangement of the inductor coil array relative to the body.

15. A system as claimed in claim 14, wherein the system comprises the seat unit and the array is fixedly mounted to the seat unit; and optionally wherein the array of coils is mounted beneath the body-facing surface.
